Europäisches Patentamt

**European Patent Office**

**Office européen des brevets**

(11) Veröffentlichungsnummer: **0 052 838**
**B1**

(12) **EUROPÄISCHE PATENTSCHRIFT**

(45) Veröffentlichungstag der Patentschrift:
**10.04.85**

(51) Int. Cl.⁴: **C 10 K 1/16,** C 10 K 1/14,
B 01 D 53/14

(21) Anmeldenummer: **81109653.6**

(22) Anmeldetag: **12.11.81**

(54) **Verfahren zum Entfernen von sauren Gasen, insbesondere Kohlendioxid, aus Gasgemischen.**

(30) Priorität: **20.11.80 DE 3043831**

(43) Veröffentlichungstag der Anmeldung:
**02.06.82 Patentblatt 82/22**

(45) Bekanntmachung des Hinweises auf die Patenterteilung:
**10.04.85 Patentblatt 85/15**

(84) Benannte Vertragsstaaten:
**BE DE FR GB IT NL**

(56) Entgegenhaltungen:
**DE - A - 2 644 323**
**DE - A - 2 759 123**
**DE - A - 2 759 124**
**DE - C - 935 144**
**DE - C - 936 716**

(73) Patentinhaber: **Linde Aktiengesellschaft,
Abraham-Lincoln-Strasse 21, D-6200 Wiesbaden (DE)**

(72) Erfinder: **Karwat, Heinz, Dr., Gistlstrasse 60,
D-8023 Pullach (DE)**

(74) Vertreter: **Schaefer, Gerhard, Dr., Linde
Aktiengesellschaft Zentrale Patentabteilung,
D-8023 Höllriegelskreuth (DE)**

ACTORUM AG

## Beschreibung

Die Erfindung betrifft ein Verfahren zum Entfernen von sauren Gasen, insbesondere Kohlendioxid, aus Gasgemischen durch Wäsche mit einer im wesentlichen aus Methanol bestehenden Waschflüssigkeit, die im Anschluss an die Wäsche regeneriert wird.

Ein derartiges Verfahren ist aus der DE-OS 27 59 123 bekannt. Bei dem dort beschriebenen Verfahren werden als physikalische Absorptionsmittel wirkende Waschflüssigkeiten, beispielsweise Methanol, zum Abtrennen von sauren Gasen aus Gasgemischen verwendet. Die Wäsche wird in Anwesenheit von Ammoniak durchgeführt, was zur Verbesserung der Wirksamkeit der Wäsche führen kann. Bezüglich der oberen Grenze für den Ammoniakzusatz wird gefordert, dass die Ammoniakkonzentration in der Waschflüssigkeit nicht mehr als 0,5 Gew-% betragen solle. Diese Forderung beruht auf der Tatsache, dass bei höheren Ammoniakkonzentrationen Festausscheidungen von Ammoniumverbindungen beobachtet wurden. Aufgrund dieser relativ geringen zugelassenen Ammoniakkonzentrationen ist es zwar möglich, mit einer relativ knapp bemessenen Waschsäule eine Feinreinigung mit geringen Restkonzentrationen an Sauergasen im gewaschenen Gas zu erhalten. Eine spürbare Verringerung des Waschmittelbedarfs, insbesondere eine Verringerung der Menge in unerwünschter Weise mitgelöster Kohlenwasserstoffe bei der Behandlung diese enthaltender Gase ist mit diesen geringen $NH_3$-Zusätzen nicht zu erreichen.

Weiterhin ist aus der DE-C 935 144 ein Verfahren zur Reinigung von Gasen bekannt, bei dem Schwefelwasserstoff und andere im Gas enthaltene Bestandteile, unter anderem niedrig siedende Kohlenwasserstoffe, unter Druck in einer oder mehreren Stufen desselben Wasch- und Tiefkühlverfahrens mit einem oder mehreren organischen, vorwiegend polaren Waschmitteln, z.B. Alkoholen, ausgewaschen werden. Dem Waschmittel kann dabei Wasser zugesetzt werden. Dies dient zur Erhöhung der Wirkung des Waschmittels, also z.B. zur verbesserten Absorption von Kohlenwasserstoffen.

Der Erfindung liegt daher die Aufgabe zugrunde, ein Verfahren der eingangs genannten Art bereitzustellen, das sich zunächst ganz allgemein durch eine verbesserte Wirtschaftlichkeit der Verfahrensführung auszeichnet. Insbesondere soll sich dies auf die unerwünschte Koabsorption von Kohlenwasserstoffen auswirken.

Diese Aufgabe wird erfindungsgemäss dadurch gelöst, dass in der regenerierten Waschflüssigkeit eine Ammoniak-Konzentration von über 0,5 Gew.%, ein Mol-Verhältnis von $NH_3$:$CO_2$ um die Zahl 2 und ein Wassergehalt von 1 bis 20 Gew.% aufrechterhalten wird.

Gemäss der Erfindung sind demnach wesentlich höhere Ammoniakkonzentrationen zulässig, als vorher angenommen worden war. Im Gegensatz zu den bisher bestehenden Befürchtungen der Fachwelt sind trotz der erfindungsgemässen Anwendung erhöhter Ammoniakkonzentrationen Feststoffausscheidungen von Ammoniumverbindungen vermeidbar. Durch die Anwesenheit einer salzartigen Verbindung aus $CO_2$ und $NH_3$ im mit Sauergas beladenen Waschmittel im Sumpf der Waschsäule wird die Löslichkeit der Kohlenwasserstoffe darin wesentlich herabgesetzt, ohne jedoch die Aufnahmefähigkeit für saure Gase, wie $CO_2$, nachteilig zu beeinflussen.

Das Mol-Verhältnis von $NH_3$:$CO_2$ in der beladenen, der Regenerierung zuzuführenden Waschflüssigkeit beträgt dabei zwei oder weniger, vorzugsweise weniger als 1, während das Mol-Verhältnis von $NH_3$:$CO_2$ in der regenerierten Waschflüssigkeit um die Zahl 2 liegt. Die regenerierte Waschflüssigkeit enthält also mit Vorteil $CO_2$ in chemischer Bindung. Weiterhin enthält das Waschmittel zusätzlich einen Wassergehalt von 1 bis 20 Gew.%.

Das Verfahren gemäss der Erfindung ist zunächst auf alle Gasgemische anwendbar, aus denen Sauergase, insbesondere Kohlendioxid, auszuwaschen sind. Als solche kommen beispielsweise Synthesegase zur Herstellung von Ammoniak oder Methanol in Frage, die etwa durch partielle Oxidation kohlenstoffhaltiger Brennstoffe gewonnen wurden. Ein wesentlicher Vorteil ergibt sich bei der Anwendung des Verfahrens auf Gasgemische, die Kohlenwasserstoffe enthalten, deren gleichzeitige Auswaschung bei Sauergasabtrennung nicht beabsichtigt ist. Es hat sich nämlich gezeigt, dass bei der Behandlung derartiger Gasgemische erhebliche Kohlenwasserstoffanteile vom Methanol aufgenommen werden, deren Abtrennung anschliessend zusätzlichen Aufwand erfordert. Durch die Anwendung des erfindungsgemässen Verfahrens auf derartige kohlenwasserstoffhaltige Gasgemische kann der Anteil an in der Waschflüssigkeit mitgelösten und ausgewaschenen Kohlenwasserstoffen nun erheblich gesenkt werden. Diese Verminderung der unmittelbar in der Waschsäule auftretenden Kohlenwasserstoffverluste liegt in der Grössenordnung von 50%, wie entsprechende Untersuchungen ergeben haben.

Mit besonderem Vorteil lässt sich die Erfindung daher bei der Reinigung von Erdgas einsetzen, das neben Methan erfahrungsgemäss immer einen gewissen Anteil an höheren, in reinem Methanol stark löslichen Kohlenwasserstoffen enthält.

Im folgenden sei die Erfindung anhand von Abbildungen näher erläutert.

Figur 1 zeigt ein Diagramm, das die Aufnahmefähigkeit ammoniakhaltigen Methanols für Kohlendioxid wiedergibt;

Figur 2 zeigt in schematischer Darstellung ein Verfahren zur Sauergaswäsche von Erdgas.

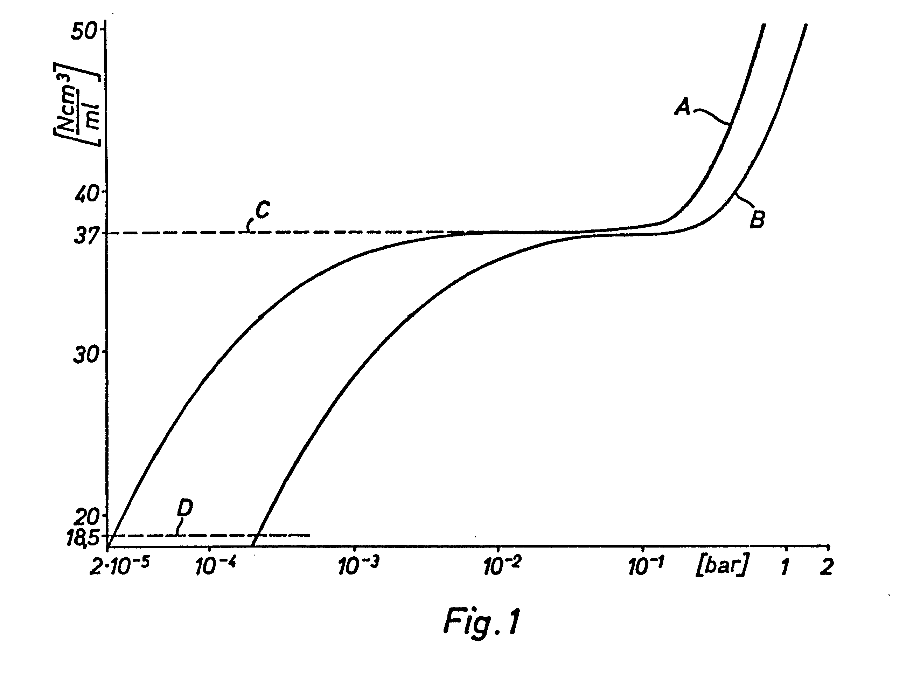

Im Diagramm der Figur 1 ist auf der Ordinate die in ammoniakhaltigem Methanol, das 37 $Ncm^3$ Ammoniak pro ml Methanol enthält, gelöste Menge an Kohlendioxid in $Nm^3$/ml Lösung, auf

der Abszisse sind die über der Lösung herrschenden Kohlendioxid-Partialdrücke in bar aufgetragen. Die Kurven A, gemessen bei –40°C, sowie B, gemessen bei –20°C, geben jeweils die Gleichgewichtsbeladungen wieder. An den Schnittpunkten der Kurven A und B mit den parallel zur Abszisse liegenden Geraden D und C befinden sich die gelösten Kohlendioxid- und Ammoniakvolumina jeweils in einem bestimmten Verhältnis, und zwar im Falle der Geraden D im Verhältnis 1:2 und im Falle der Geraden C im Verhältnis 1:1. Die Gasvolumenverhältnisse sind dabei identisch mit den Molverhältnissen, da sich beide auf Normalbedingungen beziehen. Bei den Messungen mit unteratmosphärischen Kohlendioxid-Partialdrükken wurde die Gasatmosphäre über der Lösung jeweils bis zum Gesamtdruck von 1 bar mit Stickstoff aufgefüllt.

Beim Einleiten von $CO_2$ in $NH_3$-haltiges Methanol (37 Ncm³/ml) bei –20°C fällt kurz vor Erreichen des Verhältnisses von $NH_3:CO_2 = 2$ ein Niederschlag ($NH_4NH_2CO_2$) aus, der sich bei weiterem Einleiten von $CO_2$ ($NH_3:CO_2<2$) überraschenderweise wieder auflöst. Bei einem Ammoniak-Kohlendioxid-Verhältnis von 1:1 liegt eine vollkommen klare Lösung vor. Dies könnte auf die Bildung von Carbaminsäure ($NH_3COOH$) aus jeweils äquimolaren Mengen von Ammoniumcarbamat und Kohlendioxid zurückgeführt werden. In dem Gebiet oberhalb der Geraden C ist die chemische Reaktionsfähigkeit der Lösung, bedingt durch den Ammoniakgehalt, abgesättigt. Das weitere Ansteigen der Kurven A und B in diesem Gebiet beruht nunmehr auf der physikalischen Löslichkeit des Kohlendioxids in der Lösung. Diese ist überraschenderweise für $CO_2$ ähnlich gross wie in reinem Methanol.

Der Feststoffanteil in der Lösung nimmt im Bereich zwischen den Geraden D und C mit zunehmendem Kohlendioxidgehalt ab und verschwindet bereits vor Erreichen der Geraden C, bedingt durch die Löslichkeit des Ammoniumcarbaminats in der Lösung.

Bei 27 Ncm³ $NH_3$/ml Methanol und weniger fand keine Bildung von Niederschlag beim Einleiten von $CO_2$ mehr statt.

Ein Wassergehalt im $NH_3$-haltigen Methanol kann die Niederschlagsbildung verhindern. Der dazu nötige Wassergehalt steigt mit dem $NH_3$-Gehalt im Methanol an. Bei 10 Gew.% Wasser im Methanol und einer Temperatur von –20°C bleibt bis knapp 36 Ncm³ $NH_3$/ml Methanol die Niederschlagsbildung aus. Gleichzeitig geht aber in solchem wasserhaltigen Methanol die $CO_2$-Löslichkeit im Bereich oberhalb der Linie C (physikalisch gelöstes $CO_2$) mit steigendem Wassergehalt zurück, so dass ein Wassergehalt von 20 Gew-% nicht überschritten werden soll.

Bei den in Figur 1 wiedergegebenen Messungen betrug der Ammoniakgehalt des Methanols beispielsweise 37 Ncm³/ml. Dies entspricht in etwa einem Anteil von 3,5 Gew.%. Der bisher für höchstzulässig gehaltene Wert von 0,5 Gew.% (DE-OS 27 59 123) wird somit erheblich überschritten. Dabei ist auch der hier genannte Wert von 37 Ncm³/ml Methanol noch nicht als Grenze zu betrachten. Vielmehr spielt hinsichtlich des Höchstwertes das Temperaturniveau der Wäsche und die Übertragung der Lösungs- und Reaktionswärme eine ausschlaggebende Rolle.

Durch Wäsche mit einem mit Kohlendioxid vorbeladenem, ammoniakhaltigen Methanol können hohe Reinheiten bezüglich der Kohlendioxidabtrennung erzielt werden. So geht aus den Kurven A und B hervor, dass der Kohlendioxid-Partialdruck über der der Figur 1 zugrundeliegenden, speziellen Waschflüssigkeit, wenn diese beispielsweise mit 36 Ncm³ Kohlendioxid pro ml Methanol vorbeladen ist, bei –20°C noch ca. $10^{-2}$ bar, bei –40°C sogar nur noch ca. $10^{-3}$ bar beträgt. Dies entspricht der am Kopf einer Waschsäule oberhalb der Zuspeisungsstelle der vorbeladenen Waschflüssigkeit maximal erzielbaren Reinheit im gewaschenen Gas.

Nach einer Vorbeladung der aus ammoniakhaltigem Methanol bestehenden Waschflüssigkeit mit Kohlendioxid, wobei diese Vorbeladung durch einen Punkt im Bereich zwischen den Geraden C und D repräsentiert wird, kann diese Flüssigkeit ohne Gefahr von Festausscheidungen von Carbamat zwischen Waschsäule und Regeneriersäule zirkulieren, wobei das Molverhältnis von $NH_3:CO_2$ zwischen Beladung und Regenerierung um den Wert 1 pendelt. Der untere Grenzwert wird durch den $CO_2$-Partialdruck im zu behandelnden Gas bestimmt. Somit braucht die Regenerierung des Waschmittels nicht mehr mit der gleichen Vollständigkeit durchgeführt zu werden, wie beim bekannten Verfahren. Vielmehr wird die Regenerierung nur bis zu einem bestimmten Punkt vorangetrieben, so dass ständig eine gewisse Konzentration an Kohlendioxid auch in der regenerierten Waschflüssigkeit erhalten bleibt. Die Regenerierung kann mit Vorteil in Anlehnung an die Arbeitsweise des DE-PS 843 545 durch Strippen mit einem Hilfsgas ohne vorherige Anwärmung der beladenen Waschflüssigkeit erfolgen, oder durch Kombination der Warm- und Kaltregenerierung.

Als besonderer Vorteil der Verwendung einer über 0,5 Gew.% Ammoniak enthaltenden methanolischen Waschflüssigkeit erweist sich die Tatsache, dass das Lösungsvermögen gegenüber leichteren Kohlenwasserstoffen im Vergleich zu dem reinem Methanols auf etwa die Hälfte zurückgeht. So wurde z.B. für die Löslichkeit von Propan als Modellkohlenwasserstoff in Methanol, das 2,2 Millimol $CO_2$ und 1,93 Millimol $NH_3$ pro Gramm Lösung enthielt, ein Wert von 0,65 Millimol (14,6 Ncm³) Propan/Gramm·bar gemessen, während sich in reinem Methanol 30 Ncm³ Propan/Gramm·bar lösen. Da die gesamte Waschflüssigkeitsmenge aufgrund des zusätzlichen chemischen Absorptionsvermögens des Ammoniaks für Kohlendioxid sowieso reduziert werden kann, sinken die Verluste an mitgelösten Kohlenwasserstoffen im Ergebnis um mehr als die Hälfte.

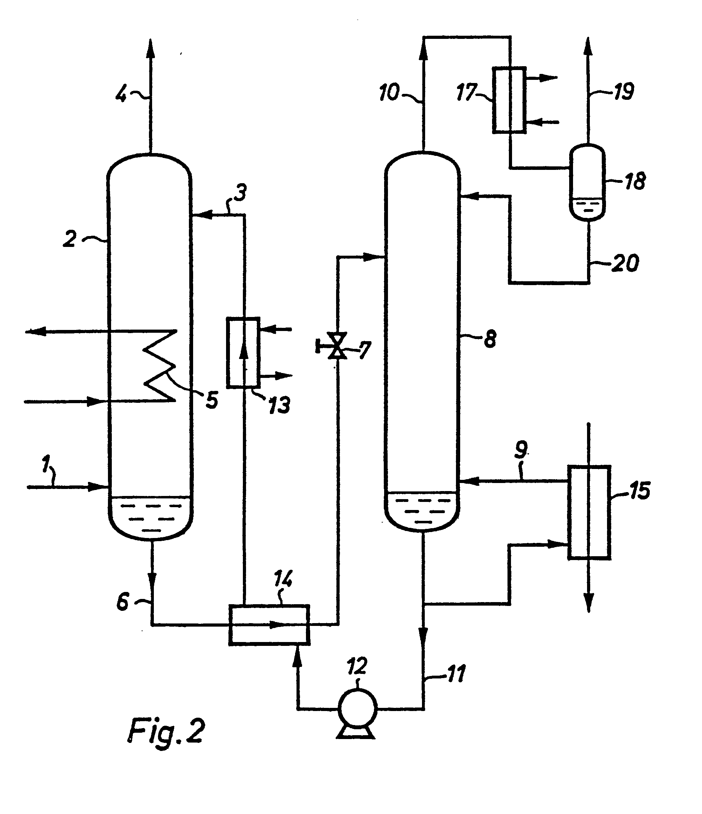

Figur 2 zeigt auf schematische Weise ein Verfahren zum Auswaschen von Sauergasen, insbesondere Kohlendioxid, aus Erdgas, bei dem nicht vollständig von $CO_2$ befreites, $NH_3$-haltiges Methanol als Waschflüssigkeit verwendet wird.

Durch eine Leitung 1 werden 10 000 Nm$^3$/h Erdgas mit 3,5 Vol.% $CO_2$, auf –20°C abgekühlt und unter einem Druck von 40 bar herangeführt. Das Erdgas steigt in einer Waschsäule 2 im Gegenstrom zu vom Kopf der Säule herablaufender, durch Leitung 3 eingespeister Waschflüssigkeit auf, wobei es bis auf einen Restgehalt von <10 ppm $CO_2$ gereinigt wird. Es verlässt die Säule durch Leitung 4.

Die Waschflüssigkeit (17,5 m$^3$/h) besteht an ihrer Einspeisestelle in die Säule 2 im wesentlichen aus Methanol und enthält 37 Nm$^3$ $NH_3$ und 27 Nm$^3$ $CO_2$/m$^3$ Waschflüssigkeit. Die Temperatur an dieser Stelle beträgt –40°C. Die Waschflüssigkeit rieselt in der Waschsäule 2 über eine Anzahl Austauschböden, an deren Stelle auch eine Füllkörperschüttung verwendet werden kann, nach unten und reichert sich dabei mit $CO_2$ sowie mit weiteren, im Erdgas eventuell vorhandenen Sauergasen, wie $H_2S$, an.

Die Absorptionswärme führt zu einer Erwärmung des Waschmittels, das seinerseits durch indirekten Wärmeaustausch die Wärme an im Kühler 5 verdampfendes Kältemittel abgibt.

Im Sumpf der Säule 2 sammelt sich beladene Waschflüssigkeit an, die auch noch einen gewissen Anteil an mitgelösten Kohlenwasserstoffen enthält. Die Temperatur der beladenen Waschflüssigkeit beträgt ca. –20°C, wobei der Temperaturanstieg auf der nicht vollständig abgeführten Absorptionswärme beruht. Der Kohlendioxidgehalt der beladenen Waschflüssigkeit beträgt nunmehr ca. 47 Nm$^3$/m$^3$ Methanol.

Durch eine Leitung 6 gelangt die beladene Waschflüssigkeit vom Sumpf über einen Wärmeaustauscher 14 und ein Ventil 7 auf die Regeneriersäule 8, in der ein Druck von 1,15 bar und eine Temperatur von ca. 67°C herrschen. In der Regeneriersäule 8 wird die beladene Waschflüssigkeit im Gegenstrom zu durch Leitung 9 eintretendem, im Verdampfer 15 erzeugtem Methanoldampf von $CO_2$ befreit. Das $CO_2$ steigt zusammen mit dem Methanoldampf auf und wird im oberen Abschnitt der Säule von herabrieselndem Methanolkondensat von $NH_3$-Spuren befreit. Durch Leitung 10 gelangt der $CO_2$-Methanoldampf in einen Kondensator 17, wo der Methanoldampf kondensiert wird und schliesslich in den Abscheider 18. Das Methanolkondensat wird durch Leitung 20 in die Säule 8 zurückgeführt. $CO_2$, eventuell mit weiteren Sauergasen, verlässt den Apparat durch Leitung 19.

Das von der vorher aufgenommenen Menge $CO_2$ befreite Lösungsmittel verlässt die Regeneriersäule 8 durch Leitung 11 und wird durch Pumpe 12 in den Wärmetauscher 14 gefördert, wo es auf etwa –17°C abgekühlt wird. Von dort strömt es durch den Kühler 13 abgekühlt auf –40°C zurück zur Säule 2.

Wird in derselben Einrichtung reines Methanol benutzt, so wird davon ein Umlauf von 28 m$^3$/h benötigt.

**Patentansprüche**

1. Verfahren zum Entfernen von sauren Gasen, insbesondere Kohlendioxid, aus Gasgemischen durch Wäsche mit einer im wesentlichen aus Methanol bestehenden Waschflüssigkeit, die im Anschluss an die Wäsche regeneriert wird, dadurch gekennzeichnet, dass in der regenerierten Waschflüssigkeit eine Ammoniak-Konzentration von über 0,5 Gew.%, ein Mol-Verhältnis von $NH_3$:$CO_2$ um die Zahl 2 und ein Wassergehalt von 1 bis 20 Gew.% aufrechterhalten wird.

2. Anwendung des Verfahrens nach Anspruch 1 zur Abtrennung von sauren Gasen aus Kohlenwasserstoffe enthaltenden Gasgemischen.

3. Anwendung des Verfahrens nach Anspruch 1 zur Abtrennung von sauren Gasen aus Erdgas.

**Claims**

1. A method of removing acid gases, particularly carbon dioxide, from gas mixtures, by washing with a washing liquid which basically consists of methanol and which is regenerated after the wash, characterised in that, an ammonia concentration of above 0.5 weight %, a mol. ratio of $NH_3$:$CO_2$ of about 2, and a water content of 1 to 20 weight %, are maintained in the regenerated washing liquid.

2. The use of the method as claimed in Claim 1 for separating acid gases from gas mixtures which contain hydrocarbons.

3. The use of the method claimed in Claim 1 for separating acid gases from natural gas.

**Revendications**

1. Procédé pour extraire des gaz acides, notamment du gaz carbonique, de mélanges gazeux par lavage avec un liquide de lavage se composant essentiellement de méthanol et qui est régénéré après le lavage, caractérisé en ce que l'on maintient dans le liquide de lavage régénéré une concentration en ammoniac supérieure à 0,5% en poids, un rapport molaire $NH_3$/$CO_2$ égal à environ 2 et une teneur en eau de 1 à 20% en poids.

2. Utilisation du procédé selon la revendication 1, pour séparer des gaz acides de mélanges gazeux contenant des hydrocarbures.

3. Utilisation du procédé selon la revendication 1, pour séparer des gaz acides du gaz naturel.

Fig.1

Fig.2